# EUROPEAN PATENT APPLICATION

(11) **EP 1 977 756 A1**
(43) Date of publication of application: **08.10.2008**
(21) Application number: 07300903.7
(22) Date of filing: 28.03.2007
(51) Int. Cl.: A61K 36/02, A61K 36/03, A61P 29/00

(54) **Algae extract containing polyphenols**

(71) Applicant: Diana Naturals, 35560 Antrain (FR)
(72) Inventor: Besnard, Matthieu, 35340 Liffre (FR); Megard, Denis, 35460 St-Brice-en-Cogles (FR); Inisan, Claude, 35830 Betton (FR); Rousseau, Isabelle, 35460 Selle-en-Cogles (FR); Lerat, Yannick, 22620 Ploubazlanec (FR); Mitjavila, Maria Teresa, 17430 Santa Coloma de Farners, Girona (ES); Simonetti, Paolo, 20145 Milano (IT); Grandieu, Patricia, Pueblo Colorado 81008 (US)
(74) Representative: Dossmann, Gérard

(57) **Abstract**

The present invention relates to an algae extract containing a high quantity of polyphenols.

The present invention also discloses a composition based on this algae extract rich in polyphenol compounds, for its use as a medicinal product intended to be used in the inflammation processes.

## Description

The present invention relates to an algae extract comprising a high quantity of polyphenols.
The present invention also discloses a composition based on this algae extract rich in polyphenol compounds, for its use as a medicinal product intended to be used in the inflammation processes.

Polyphenols are a group of secondary metabolites widely represented in plants and form a family of compounds which are very diverse in terms of structure or size. In the phylum of brown algae (Phaeophyceae), the phenolic compounds or phlorotannins are derived by polymerization from one and the same monomer: phloroglucinol (1,3,5-trihydroxybenzene) (Formule 1).

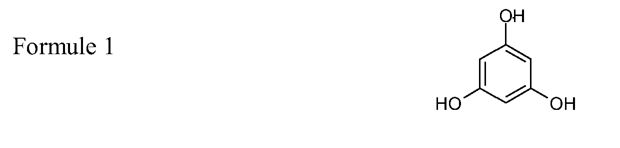

Various types of phlorotannin exist which are distinguishable by the number of monomeric units and their type of bonds:
- fucols composed of phloroglucinol monomers linked by aryl-aryl bonds.
- Phlorethols composed of monomers linked by aryl-ether bonds in the para, ortho or meta position.
- Fucophlorethols, highly represented in brown algae, consisting of numerous phloroglucinol monomers attached by aryl-aryl and aryl-ethyl bonds.
- Hydroxyfucophlorethols, not widely represented in this phylum, consist of fucophlorethols with one or more additional hydroxyl groups.
   Other less widespread algal polyphenols exist such as fuhalols, phlorotannins which are the most sensitive to oxidation, hydroxyfuhalols, dehydroxyfuhalols, isofuhalols, pseudofuhalols, carmalols, eckols and sulphated phlorotannins.
   *Fucus vesiculosus* and *Ascophyllum nodosum* are the two species of brown algae which are richest in phlorotannins with contents which vary between 3 and 10% (dry weight) according to the season, the period of harvest, the geographical origin, the level of irradiation and the stress inflicted by environment and salinity (Connan, S., et al., 2004, Botanica Marina, 47, pp410-16).
   Algal phlorotannins differ from the tannins derived from terrestrial plants in their biosynthesis pathway. In brown algae, the low-molecular weight polyphenols appear to have antifungal, antiviral, antibiotic and antialgal properties. Other studies report a phlorotannin toxicity to a variety of marine animals. Polyphenols are thought to be very potent chelators of heavy metals and are thus thought to participate in the detoxification of cells. They are also thought to play a protective role against UV radiation. Finally, phlorotannins have a high antioxidant power. Polyphenols of high molecular weight (between 30 000 and 100 000 daltons), contained in the crude extract of *Fucus vesiculosus, Ascophyllum nodosum* and *Pelvetia canaliculata* are thought to be potent inhibitors of enzymes involved in oxidative stress (alpha-amylase, lipase and trypsin).

The use of algae extracts for the preparation of pharmaceutical, cosmetic or food compositions, or of compositions for agricultural use, is known from the document WO 91/07946 (SECMA). However, this document does not describe the composition of the algae extracts obtained.

The applicant has developed novel algae extracts with a high content of phenolic compounds, which constitute the subject of the invention.
Another subject is the application of these algae extracts as medicament.
Another subject of the invention consists of the use of this extract in pharmaceutical composition for inflammation processes.
The inflammation processes are at least implicated in arthritis and allergy.
The subject of the invention is also the process for producing this extract.
Other subjects will emerge on reading the description and the examples and drawings which follow.

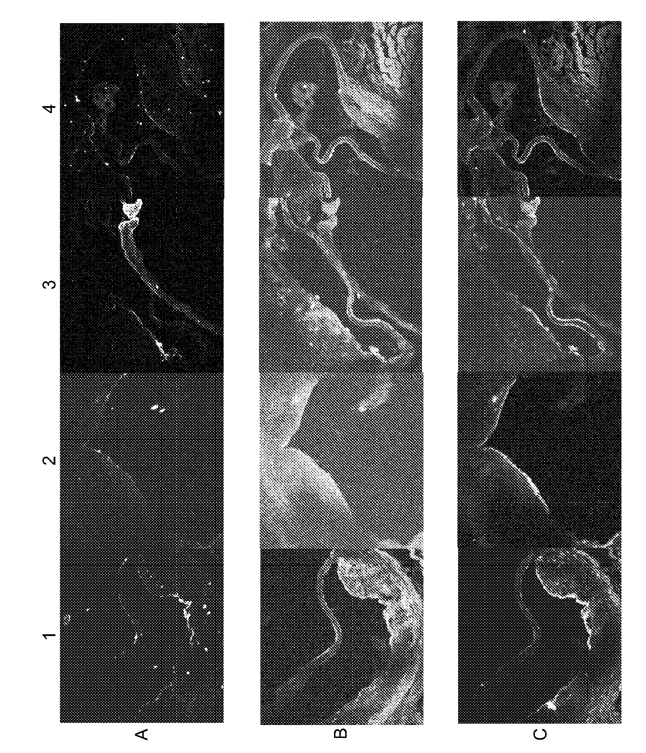

FIG. 1.- Representative image of the expression of cellular adhesion molecules *(A:* P-Selectin; *B:* VCAM-1; C: ICAM-1) in the endothelium (1 - Control group; 2 - Aqueous extract; 3 - Ethanolic extract; 4 - Phloroglucinol). The more dense fluorescence granules are secondary antibodies precipitates.

The polyphenolic extract in accordance with the invention comprises between 11 and 19 % polyphenols by weight relative to the dry weight of the algae extract, and preferably at least 15 % of polyphenols by weight relative to the dry weight of the algae extract. The content of phenolic compounds of an algae extract according to the invention is estimated by means of the reference "Folin Ciocalteu" assay method, according to Glombitza.
The polyphenolic extract in accordance with the invention contains between 0 and 1 % by weight of iodine relative to the dry weight of the extract.
The recommended nutrient intake (RNI) of iodine depends on the geological context, sex, age and physical and pathological conditions:

| | RNI in Europe (µg/day) |
|---|---|
| Children 6 months - 6 years | 90 |
| Children 7-10 years | 120 |
| Adolescents and adults | 150 |
| Pregnant and breast-feeding women | 200 |

Source: Maretin et al., 2001. Apports nutritionnels conseillés pour la population française (Recommended nutrient intake for the French population). 3rd ed.

In pregnant women, the RNI takes into account an increased renal clearance of iodine during pregnancy and the specific needs of the foetus. In breast-feeding women, the RNI compensates for the average daily losses of 30 to 50 µg of iodine in breast milk.
There is no consensus on the iodine toxicity threshold. The value of 1000 to 2000 µg/day, corresponding to the intake observed for the onset of the Wolff-Chaikoff effect, a warning sign for thyroid disorders, appears as a pathological threshold.

Regarding iodine content in food, the European Scientific Committee on Food (SCF) recently gave an opinion on the maximum upper limits for vitamins and minerals which can pose a risk to health. Iodine was reviewed (Opinion of the Scientific Committee on Food on the Tolerable Upper Intake Level of Iodine, of 26 September 2002) and it is stated that the tolerable upper intake is 600 µg/day. It is also specified that in France, in the regions where disorders due to an iodine deficiency have been present for a long time, it is suggested not to exceed an upper threshold of 500 µg/day in order to avoid the risk of hyperthyroidism appearing. In the United States, this limit, set by the JECFA, is 1000 µg/day (Gévaudan, 2000).

Regarding food supplements, anew regulation was set by EU (2006/352) listing maximal daily amounts for a series of compounds including iodine. A French regulation was issued to comply this the EU directive and was published in JORF 123 the 09^{th} of May 2006. This regulation sets the maximal daily amount of iodine brought by food complement as 150 µg/day. Maximum value for daily intake from normal food remains enchanged.
To comply with this recommended iodine intake, the iodine content in milligram per gram of dry weight is reduced compared with the initial concentration in the raw material used. The final iodine concentration in the extract is still less than 1% of the dry weight of the alga extract.

The algae extract according to the invention may be obtained from brown algae, preferably from food macro- or microalgae.
These algae may be of the Phaeophyceae family, and more preferably of the *Fucus sp.* or *Ascophyllum sp.* species. The algae extract according to the invention may be obtained from a mixture of algae of this family.

The subject of the invention is the use of this algae extract rich in polyphenols for the preparation of a medicament directed to inflammation processes, leading to at least arthritis and allergy.

Inflammation is the complex host systemic and local response to injury infection. Clinically the cardinal features of inflammation are heat, redness (erythema), swelling (oedema), and pain, resulting in loss of function. The host response attempts to limit the extent of damage, counteract infection and promote healing and recovery of function.
Inflammation has two main components - cellular and exudative.
The cellular component involves the movement of white blood cells from blood vessels into the inflamed tissue. The white blood cells, or leukocytes, take on an important role in inflammation; they extravasate from the capillaries into tissue, and act as phagocytes, picking up bacteria and cellular debris. They may also aid by walling off an infection and preventing its spread.
The exudative component involves the movement of fluid, usually containing many important proteins such as fibrin and immunoglobulins (antibodies). Blood vessels are dilated upstream of an infection (causing redness and heat) and constricted downstream while capillary permeability to the affected tissue is increased, resulting in a net loss of blood plasma into the tissue - giving rise to edema or swelling. The swelling distends the tissues, compresses nerve endings, and thus causes pain. Inflammation can be recognized by nitric oxide.
If inflammation of the affected site persists, released cytokines IL-1 and TNF will activate endothelial cells to upregulate receptors VCAM-1 (vascular cell adhesion molecule-1), ICAM-1 (intercellular adhesion molecule-1), E-selectin, and L-selectin for various immune cells. Receptor upregulation increases extravasation of neutrophils, monocytes, activated T-helper and T-cytotoxic, and memory T and B cells to the infected site.
If the injurious agent continues, chronic inflammation will ensue. This process, marked by inflammation lasting many days, months or even years, may lead to the formation of a chronic wound. Chronic inflammation is characterised by a dominating presence of macrophages in the injured tissue, which extravasate via the same methods discussed above (ICAM-1 VCAM-1). These cells are powerful defensive agents of the body, but the toxins they release (including reactive oxygen species) are injurious to the organism's own tissues as well as invading agents. This is why chronic inflammation is almost always accompanied by tissue destruction.

The pharmaceutical compositions comprising a polyphenolic algae extract according to the invention can be administered to humans by ordinary routes, for example, orally. The pharmaceutical compositions according to the invention can be used as such or integrated in a food matrix. Dose and time of administration vary depending upon administration routes, condition of disease, body weight, etc... The compositions can be administered to adult in a daily dose once or in several portions.
According to this mode of administration, the composition according to the invention may be provided in any of the forms normally used in pharmaceutical products. The compositions may be provided in particular in the form of powders, granules, tablets, pills, capsules, , etc.
The pharmaceutical compositions can be prepared in a conventional manner, using conventional carriers for preparations. That is, in the case of preparing oral preparations, excipients or carriers are added to the active ingredient and if necessary, binders, disintegrators, lubricants and coloring agents are further added thereto and then prepared into tablets, granules, powders, capsules, etc....
The medicament comprising polyphenolic algae extract composition according to the invention is directed to inflammation processes.

The method allowing the extraction and the selective purification of the phenolic compounds from algae is characterized in that it comprises the following steps:
- The ground algae are subjected to one or more solid/liquid extractions in the presence or absence of added water.
- The liquid phase is then separated from the solid phase for example by centrifugation.
- Optionally, filtration of the mixture is carried out.
- The liquid thus obtained, free of solid residue, is optionally purified by a membrane filtration and/or chromatography technique in order to remove part of the iodine and to concentrate the polyphenols of interest.
- In the case of the chromatographic purification technique, the liquid is loaded onto a column filled with adsorbent resin.
- After washing, elution is performed with an aqueous-alcoholic solution containing between 20 and 90% of alcohol by weight and preferably between 50 and 80% of alcohol.
- The extract thus obtained is then concentrated under reduced pressure at a temperature of less than 80°C, preferably less than 60°C.
- The product thus obtained is then spray-dried or freeze-dried in order to obtain a brown powder containing between 11 and 19 % of polyphenols by weight, and preferably at least 15 % of polyphenols by weight.

The algae are harvested, rapidly washed with soft water, dried at 50°C for 24 hours or 100°C for 1 hour and then finely ground (120-250 µm).
An aqueous extraction is performed in order to solubilize the polyphenols and the iodine at room temperature with an algae content in the water of 2.5 to 5%, with mechanical stirring for 4 hours. The aqueous extraction may be reduced if fresh algae are used as starting materials.

After filtration on a filter press, the solid residue is separated from the aqueous extract. The aqueous extract undergoes a first purification step in order to remove the alginates by precipitation of the alginates in the presence of an excess of calcium chloride. After filtration, the aqueous extract undergoes a second purification step by diafiltration on a 1000 dalton membrane in order to remove the iodine and the low-molecular weight compounds. The retentate is preserved. The purified aqueous extract is dried either by freeze-drying or by spray-drying.
The extract may be obtained by the method described above, preferably from food algae, advantageously derived from the *Fucus sp.* and *Ascophyllum sp.* species.
This food algae extract possessing the characteristics stated, being possibly obtained according to the method described above, can be used for the preparation of medicaments for prevention of inflammation processes, particularly in case of arthritis and allergy.

The following examples illustrate the invention without at all limiting it.

### Example 1: Production of an extract of brown algae

Brown algae *Fucus sp.* and/or *Ascophyllum sp.* are harvested and then dried at 100°C for 1 hour. They are then ground so as to obtain a range of particles from 0.3 to 1 cm.
An aqueous extraction at acidic pH is then performed in the hot state, at a temperature greater than 80°C, at a pH between 1 and 1.5 obtained by acidification with sulphuric acid, for at least 2 hours, with an algae content in the acidic solution of 5 to 20%. After pressing in order to separate the solid residue from the liquid extract, this extract is ultrafiltered in order to remove the insolubles therefrom. The permeate is then concentrated under vacuum, at a temperature of less than 70°C, in order to reduce the volume of the extract.
The purification is performed on a column containing an adsorbent resin. After loading the extract, the resin is washed with purified water in order to remove the impurities. The polyphenols are then eluted with an aqueous-alcoholic solution containing between 20 and 80% of ethanol by weight.
The eluate is then concentrated under reduced pressure, at a temperature of less than 50°C. The liquid extract thus obtained is then spray-dried or freeze-dried in order to obtain a brown powder rich in polyphenols, the extract CEV-ETE1

### Example 2: Assay of the phenolic compounds on the extract CEV-ETE1

This assay of the phlorotannins is carried out by the Folin Ciocalteu method according to Glombitza. The principle of this method is based on the oxidation of phenols in the presence of phosphomolybdic and phosphotungstic acids, leading to the formation of blue compounds ("molybdenum blue" and "tungsten blue"), the absorption maximum of which is at 760 nm.
A calibration series is prepared from phloroglucinol (Merck, No. 107069, CAS No. 108-73-6) with concentrations of 0, 40, 60, 80, 100 and 120 mg/L. To 2 ml of these different phloroglucinol solutions were added 10 ml of water, 12 ml of Na₂CO₃ (29% m/v) and 1 ml of Folin-Ciocalteu reagent (Merck, No. 109001). The absorbance was then read at 760 nm (Perkin Elmer Lambda UV-VIS spectrophotometer) after 30 minutes of reaction in the dark.
50 mg of dry extract CEV-ETE1 were dissolved in 25 ml of water. 10 ml of this solution were then diluted with 15 ml of water, constituting the solution to be assayed. 2 ml of this solution were then treated. Serial dilutions were then made in order to obtain an absorbance of between 0.3 and 0.8. The results obtained correspond to the total polyphenols and are expressed as phloroglucinol equivalent.
For the normal algae (fucus), the preparations of solutions have been made based on the same protocol.

| | Normal alga | Extract CEV-ETE1 |
|---|---|---|
| Total polyphenols phloroglucinol eq.(Folin Ciocalteu method), in % by dry weight | 6 | 19 |

### Example 3: Method for assaying iodine in the powdered extract CEV-ETE1:

The method for assaying iodine in algae and products derived from algae is extracted from the Food Chemical Codex III.
Briefly, the algal material undergoes dry alkaline mineralization in the hot state. The iodides are then extracted and then oxidized to iodate with bromine. The iodates are reduced to molecular iodine which is then titrated with the aid of sodium thiosulphate.

| | Normal alga | Extract CEV-ETE 1 |
|---|---|---|
| Iodine FCC III method in mg/kg | 756 | 307 |

### Example 4 : Evaluation of antioxidant blood vitamins by in vivo study in healthy human

Forty healthy human volunteers were divided into two groups and assigned to the polyphenolic extract [500 mg (4 capsules) of polyphenolic extract CEV-ETE1] or placebo intake in a crossover design for 6 weeks (6 weeks of wash-out between the treatments).

| Treatment | 6 weeks | 6 weeks | 6 weeks |
|---|---|---|---|
| Group A (n=20) | Extract | Wash-out | Placebo |
| Group B (n=20) | Placebo | Wash-out | Extract |

Blood samples were collected from each participant before intake and 3, 9, 16, 21 and 42 days after ingestion.

### a) Determination of Vitamin C in plasma

A standard solution comprising 50mg of Ascorbic acid (SIGMA cod. 2174) was diluted in 50ml of MPA at 1%. The standard solution was diluted at 1:50, 1:100, 1:200 and 1:400. The concentrations obtained were the followings: 2.5µl/ml, 5µl/ml, 10µl/ml, 20µl/ml
300µl of the fresh plasma were immediately transferred in an Eppendorf tube (1ml) which contained 300µl of metaphosphoric acid (MPA, 10%). The tube was stored at -80°C until HPLC analysis. After being thawed the samples were centrifuged at 10000 x g for 1 minute. The supernatant was filtered and the obtained sample was used for HPLC analysis (Atlantis dC18 250mm x 4.6mm i.d.).

### Vitamin C plasma levels as mean±SE (µmol/ml)

| Days | 0 | 21 | 42 |
|---|---|---|---|
| Placebo (n=20) | 54.2±6.5 | 52.5±8.1 | 56.4±4.6 |
| CEV-ETE1 (n=20) | 56.1±6.2 | 61.8±5.5^{a} | 66.6±4.9^{a} |

| | | | |
|---|---|---|---|
| a : Significantly different vs Placebo | | | |

The levels of plasma vitamin C were significantly higher after 21 and 42 days of supplementation with the Fucus Extract CEV-ETE1 compared with a normal algae powder.

### b) Determination of Vitamin A and E in plasma

Standard solution comprises 50mg of α-tocopherol or retinol (SIGMA) were diluted in 50ml of absolute ethanol. Solutions used for injection were diluted at 1:50, 1:100, 1:200 and 1:400. and the concentrations obtained were checked spectrophotometrically to 292 and 325 nm, respectively.
100 µl of fresh plasma were immediately transferred in dark vials (1 ml). The vials were vortexed for 10 sec, added with 200 µl of n-hexane and subsequently vortexed for 40 sec. The samples were centrifuged at 1000 x g for 5 minute. 150 µl of supernatant was transferred in a dark vial (2 ml), evaporated with nitrogen and diluted in 100 µl of methanol: tetrahydrofuran (950:5 v/v).

### Vitamin A plasma levels as means±SE (µg/ml)

| Days | Placebo (n=20) | Fucus Extract CEV-ETE1 (n=20) |
|---|---|---|
| 0 | 0.57±0.02 | 0.56±0.01 |
| 3 | 0.60±0.04 | 0.54±0.02 |
| 9 | 0.61±0.05 | 0.57±0.02 |
| 12 | 0.58±0.09 | 0.55±0.02 |
| 21 | 0.55±0.04 | 0.51±0.03 |
| 42 | 0.57±0.1 | 0.54±0.08 |

The levels of plasma vitamin A were not affected by the supplementation of Fucus Extract CEV-ETE1.

### Vitamin E plasma levels as means±SE (µg/ml)

| Days | Placebo (n=20) | Fucus Extract CEV-ETE1 (n=20) |
|---|---|---|
| 0 | 8.82±0.57 | 8.23±0.49 |
| 3 | 8.80±0.92 | 8.34±0.34 |
| 9 | 8.37±0.55 | 8.76±0.79 |
| 12 | 9.54±0.99 | 8.45±0.25 |
| 21 | 8.6±0.48 | 8.12±0.67 |
| 42 | 8.44±1.28 | 8.07±0.50 |

The levels of plasma vitamin E were not affected by the supplementation of Fucus Extract CEV-ETE1.

### c) Determination of α-tocopherol in red blood cells

Standard solution comprises 50mg of α-tocopherol (SIGMA) diluted in 50ml of absolute ethanol. Solutions used for injection were diluted at 1:50, 1:100, 1:200 and 1:400. The concentrations obtained were checked spectrophotometrically to 292 nm.
Extraction and HPLC determination of red blood cells α-tocopherol were performed as described by Hatam and Kayden (Hatam, L.J.; Kayden, H.J. J. Lipid Res., 1979, 20, 639-645), with the following adjustments: 0.3 g of red blood cells, separated from whole blood by centrifugation at 10000 x g for 1 min and washed twice with an equal volume of a physiologic solution (NaCl 0.9% w/v), was weighed in 10 mL plastic tubes. Five hundred µL of Triton X-100 (1% w/v in water), 500 µL of distilled water, and 1 mL of ascorbic acid (1% w/v in ethyl alcohol) were added. After 10s vortexing, an ultrasonic bath was employed for 5 min to perform cells lyses. Four mL of hexane was added and the tubes vortexed for 10 min. After centrifugation at 800 x g for 10 min, 3.5 mL of the supernatant was transferred into 10 mL clean tubes containing 50 µL of BHT (0.1% w/v in hexane). 2.5 mL of hexane was added to the tubes containing RBC to perform a second extraction by vortexing for 10 min, and after centrifugation at 800 x g for 10 min, 2 mL of the supernatant was transferred into the corresponding tube. The whole extraction solvent was evaporated under vacuum and nitrogen flow, then 500 µL of hexane was added, the tubes vortexed for 10 sec and the content transferred into a glass vial.
For the HPLC analysis, a Model 9001 (Varian, Cary, Australia) pump connected to a Ultra Wisp 715 (Waters, Milford, MA, USA) autosampler and to a LC 240 fluorescence detector (Perkin-Elmer, Beaconsfield, UK) was used. Chromatogram integration was obtained with a Millennium workstation (Waters). Separations were performed on a 250 x 4.6 mm i.d. LiChrosorb Si60 column, (Merck, Darmstadt, Germany), using hexane-ethyl acetate (1000:75, v/v) as mobile phase. The flow-rate was 2 mL/min. Fluorescence detector was set at 290 nm in excitation and 330 nm in emission. Calibration curves were obtained by injecting standard α-tocopherol, and linearity was assessed in the range 0.8-8 µg/mL.

### Red blood cells levels of α-tocopherol as means±SE (µg/g)

| Days | Placebo (n=20) | Fucus Extract CEV-ETE1 (n=20) |
|---|---|---|
| 0 | 2.07±0.04 | 2.06±0.08 |
| 21 | 2.08±0.04 | 2.05±0.05 |
| 42 | 2.15±0.09 | 2.12±0.06 |

The levels of plasma α-tocopherol were not affected by the supplementation of Fucus Extract CEV-ETE1.

### Example 5 : Evaluation of inflammation by in vivo study in healthy rats

Healthy rats received (included in a semisynthetic diet) 200 mg/kg/day body weight of Fucus Extract CEV-ETE1 or 43 mg/kg body weight of phloroglucinol for 4 weeks. Results were compared to a control group.
At the end of the feeding period, rats were anesthetized with isofluorane and exsanguinated. Heparin was used as anticoagulant for blood collection. Plasma was obtained following blood centrifugation at 1,770 g at 4°C for 15 min. The thoracic aorta were extracted and cut in segments as previously described (López et al., 2001).
The evolution of body weight of these healthy rats has been monitored.

### Evolution of healthy rat body weight (in grams) along treatment. Data are means ± SEM, n = 8

| | Week | | | | |
|---|---|---|---|---|---|
| | 0 | 1 | 2 | 3 | 4 |
| Control | 199 ± 8 | 198 ± 6 | 242 ± 5 | 273 ± 6 | 299 ± 8 |
| Fucus Extract CEV-ETE1 | 201 ± 9 | 206 ± 7 | 241 ± 5 | 271 ± 6 | 295 ± 7 |
| Phloroglucinol | 193 ± 11 | 186 ± 9 | 220 ± 7 | 248 ± 6 | 270 ± 6 |

### a) Modulation of superoxide(O₂⁻) and nitric oxide (NO) production by aorta ex vivo

O₂⁻ and NO are two free radicals that exert regulatory and inflammatory functions in the vascular tissue. The O₂⁻ production by aortic segments was analysed from the four groups of rats in the presence of a NADPH-like cofactor and coelenterazine (Tarpey et al., 1999). The NO basal production was measured in other segments by electron paramagnetic resonance (e.p.r.) (López et al., 2004) by using Fe and diethyldithiocarbamic acid (DETC) as trapping agents that transform NO to the Fe-DETC-NO complex. eNOS expression was also evaluated in rat aorta by Western blot.

### Superoxide and nitric oxide production by rat aorta after daily doses of extracts for 4 weeks.

| | Superoxide production (luminescence units/mg tissue) | Nitric oxide production (e.p.r. units/mg tissue) |
|---|---|---|
| Control | 1198 ± 21 | 452 ± 21 |
| Fucus Extract CEV-ETE1 | 1177 ± 39 | 669 ± 39*** |
| Phloroglucinol | 1156 ± 24 | 559 ± 35* |

Data are means ± SEM, n = 8. Differences of treated groups from control are indicated by **P* < 0.05, ****P* < 0.001.

Diets did not modify the O₂⁻ production but basal NO generation was increased, which is correlated with the 80% and 20% increase of eNOS expression in rats fed with the Fucus Extract CEV-ETE1. Phloroglucinol had not effect. However, the increase in NO at vascular level was not reflected by an increase of its concentration in plasma probably because the great dilution of NO.

### b) Nitrites in plasma

Plasma nitrites can be used as an indicator of the overall NO production by the organism. Nitrites are measured through a catalytical conversion system that transforms nitrites of samples to NO that was monitored by an NO electrode.

### Nitrites in plasma of rats after daily doses of extracts for 4 weeks

| | Nitrites in plasma (µmol/L) |
|---|---|
| Control | 2.86 ± 0.24 |
| Fucus Extract CEV-ETE1 | 2.99 ± 0.23 |
| Phloroglucinol | 3.06 ± 0.18 |

| | |
|---|---|
| Data are means ± SEM, n = 8. Nitrites level is not modified by the addition of Fucus extract or phloroglucinol. | |

### Example 6 : Markers of inflammation

### a) experiments on ApoE^{-/}⁻ mice.

After weaning, two groups of male ApoE^{-/-} mice (Charles River France) were fed since 4 wk of age on semipurified diets containing 0.15% cholesterol, 5% lipids and 200 or 43 mg/kg/day of the extracts or phloroglucinol, respectively that were prepared in our laboratory. Diets were prepared weekly and stored at -20°C to prevent oxidation and food was provided and removed daily. Body weight was recorded weekly.

### Evolution of apoE deficient mice body weight (in grams) along treatment. Data are means ± SEM, n = 14

| | Week | | | | | |
|---|---|---|---|---|---|---|
| | 0 | 1 | 2 | 3 | 4 | 5 |
| Control | 16 ± 0.52 | 18 ± 0.44 | 20 ± 0.43 | 22 ±0.51 | 23 ± 0.40 | 23 ± 0.43 |
| Fucus Extract CEV-ETE1 | 16 ± 0.89 | 18 ± 1.05 | 20 ± 0.90 | 22 ± 0.95 | 23 ± 1.00 | 24 ± 0.93 |
| Phloroglucinol | 15 ± 0.61 | 17 ± 0.50 | 19 ± 0.36 | 21 ± 0.41 | 23 ± 0.43 | 24 ± 0.50 |

| | Week | | | | | |
|---|---|---|---|---|---|---|
| | 6 | 7 | 8 | 9 | 10 | 11 |
| Control | 24 ± 0.51 | 24 ± 0.55 | 25 ± 0.50 | 26 ± 0.45 | 26 ± 0.57 | 26 ± 0.61 |
| Fucus Extract CEV-ETE1 | 25 ± 0.98 | 26 ± 1.02 | 27 ± 0.88 | 27 ± 0.98 | 28 ± 0.96 | 28 ± 0.93 |
| Phloroglucinol | 25 ± 0.61 | 26 ± 0.58 | 26 ± 0.70 | 27 ± 0.67 | 27 ± 0.83 | 28 ± 0.64 |

The treatment in apoE^{-/-} mice showed no signs of toxicity after any of these long treatments. The evolution of body weight is normal. These results differ from those observed in healthy rats. But the apoE(-/-) mice are not healthy and their body weight increase is smaller than for healthy Swiss mice. The doses of alga extracts ingested were about 200 mg/kg b.w. and 40 mg/kg b.w. for phloroglucinol.
At 16 week of age, mice were anesthetized with sodium urethane (1.5 mg/kg ip) and exsanguinated by cardiac puncture using heparin as anticoagulant. Plasma was obtained after centrifugation at 1770 g at 4°C for 15 min, and an aliquot was frozen at - 80°C. The procedures and the care of the mice complied with the European Union guidelines.

### b) Immunofluorescence

Seven serial sections (10 µm) of the aortic root were washed in 10 mM PBS and 20 mM glycine and blocked with 1% BSA in 10 mM PBS. In addition, two negative controls were present in all staining series. Finally, calibration of an Olympus image analyzer, for the whole study, was kept at the same original setting. The following primary polyclonal antibodies were used: anti-mouse P-selectine (Rabbit IgG, 5 µg/mL) purchased from Chemicon. Immunostains (ciutat, pais), anti-mouse VCAM-1 (Rat IgG2a, k, 10 µg/mL) purchased from eBioscience (citat, pais) and anti-mouse ICAM-1 (Goat IgG, 2 µg/mL) purchased from R&D Systems (ciutat, pais). They were visualized after incubation with the corresponding secondary antibodies (labelled Donkey Anti-Rabbit IgG 647 (1/500), labelled Donkey Anti-Rat IgG 488 and labelled Donkey Anti-Goat IgG 546) purchased from Molecular Probes (ciutat, pais).

Monocyte chemotactic protein-1 (MCP-1) plays a role in the recruitment of monocytes to sites of injury and infection. Chemokines are a family of pro-inflammatory activation-inducible cytokines, or small secreted protein signals. Chemokines induce directed chemotaxis in nearby responsive cells, hence the name chemotactic cytokines. P-selectine and vascular cellular adhesion molecules (VCAM) and intercellular adhesion molecules (ICAM) are involved in rolling and migration of monocytes to the subendothelial space, respectively.
Monocytes in the subendothelial space become macrophages. When macrophages phagocyte oxidized LDL they become foam cells that initiate the atherosclerosis process.

### Measurement of MCP-1, P-selectine, VCAM and ICAM in the aortic root from apoE-/- mice.

| | MCP-1 | P-selectine | VCAM-1 | ICAM-1 |
|---|---|---|---|---|
| Control | 185 ± 0.9 | 148 ± 22 | 184 ± 21 | 160 ± 5 |
| Fucus Extract CEV-ETE1 | 167 ± 7* | 100 ± 7 * | 170 ± 16 | 120 ± 12* |
| Phloroglucinol | 178 ± 2* | 86 ± 14* | 163 ± 1 | 135 ± 29 |

| | | | | |
|---|---|---|---|---|
| Data are means ± SEM, n = 3-4 Differences of treated groups from control are indicated by **P* < 0.05. | | | | |

The fluorescence has been evaluated only at the endothelium level of all the mice. Thus, a reduction in the previous molecules is regarded as a beneficial/preventive effect against the atherosclerosis development.

The confocal analysis by using specific antibodies for cell adhesion molecules (FIG. 1) indicates that P-selectine, VCAM-1 and ICAM-1 were reduced by treatments. These molecules are involved in rolling and extravasation of macrophages to the subendothelial space.

## Claims

1. Algae extract, **characterized in that** it comprises between 11 and 19% of polyphenols, percentage expressed by weight of polyphenols relative to the dry weight of the extract.

2. Algae extract according to Claim 1, **characterized in that** it contains at least 15% of polyphenols, percentage expressed by weight of polyphenols relative to the dry weight of the extract.

3. Algae extract according to either of Claims 1 and 2, **characterized in that** it contains between 0 and 1% by weight of iodine relative to the dry weight of the extract.

4. Algae extract according to Claims 1 to 3, **characterized in that** the said algae are food macroalgae or microalgae.

5. Algae extract according to any one of Claims 1 to 4, **characterized in that** the said algae are algae of the Phaeophyceae family.

6. Algae extract according to any one of Claims 1 to 5, **characterized in that** the said algae are of the *Fucus sp.* and/or *Ascophyllum sp.* species.

7. Algae extract according to any of Claims 1 to 6, for its use as a medicament.

8. Pharmaceutical composition comprising, in an acceptable pharmaceutical medium, an algae extract according to any of Claims 1 to 7.

9. Medicament containing at least an algae extract according to any of Claims 1 to 7 for the treatment of inflammation processes.

10. Method for the manufacture of an algae extract according to any one of Claims 1 to 7, comprising the following steps:
a) a solid-liquid extraction of the algae, dried or not, ground beforehand or not, is carried out
b) a separation of the liquid and solid phases of the mixture is carried out
c) the liquid phase is purified
d) the phase rich in polyphenols is recovered, and it is optionally concentrated and/or freeze-dried or spray-dried.

11. Method according to Claim 10, in which step b) for separating the liquid and solid phases is carried out by filtration.

12. Method according to Claim 10 or 11, in which step c) for purification of the liquid phase is carried out by a chromatographic method.

13. Use of an algae extract according to Claims 1 to 7 for the manufacture of a medicament for treatment of inflammation processes.

14. Use of an algae extract according to Claim 13 for the manufacture of a medicament for treatment of allergies

15. Use of an algae extract according to Claim 13 for the manufacture of a medicament for treatment of arthritis.
